(19) Europäisches Patentamt / European Patent Office / Office européen des brevets

(11) **EP 3 599 994 B1**

(12) **EUROPÄISCHE PATENTSCHRIFT**

(45) Veröffentlichungstag und Bekanntmachung des Hinweises auf die Patenterteilung:
**22.02.2023   Patentblatt 2023/08**

(21) Anmeldenummer: **18710486.4**

(22) Anmeldetag: **16.03.2018**

(51) Internationale Patentklassifikation (IPC):
**G16H 20/10** (2018.01)     **A61B 5/00** (2006.01)

(52) Gemeinsame Patentklassifikation (CPC):
**A61B 5/0002; A61B 5/4833; A61B 5/746; A61B 5/7465; G16H 20/10**

(86) Internationale Anmeldenummer:
**PCT/EP2018/056651**

(87) Internationale Veröffentlichungsnummer:
**WO 2018/172204 (27.09.2018 Gazette 2018/39)**

(54) **UNTERSTÜTZUNG VON PATIENTEN BEI DER WIEDERHOLTEN EINNAHME VON ARZNEIMITTELN**

ASSISTANCE OF PATIENTS WITH THE REPEATED TAKING OF MEDICINES

AIDE AUX PATIENTS POUR LA PRISE RÉPÉTÉE DE MÉDICAMENTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.03.2017   EP 17162556**
**10.10.2017   EP 17195605**

(43) Veröffentlichungstag der Anmeldung:
**05.02.2020   Patentblatt 2020/06**

(73) Patentinhaber: **Bayer Aktiengesellschaft**
**51373 Leverkusen (DE)**

(72) Erfinder:
• **TRÜBEL, Hubert**
**42281 Wuppertal (DE)**
• **BÖHME, Philip, Patrick**
**35039 Marburg (DE)**
• **VAN EICKELS, Martin**
**10115 Berlin (DE)**
• **DINH, Wilfried**
**46414 Rhede (DE)**
• **KRAMER, Frank**
**42489 Wülfrath (DE)**
• **CASTELLON, Carlos**
**14129 Berlin (DE)**

(74) Vertreter: **BIP Patents**
**c/o Bayer Intellectual Property GmbH**
**Alfred-Nobel-Straße 50**
**40789 Monheim am Rhein (DE)**

(56) Entgegenhaltungen:
WO-A1-02/086791          WO-A1-2011/097313
WO-A2-01/93762           WO-A2-2007/081947
WO-A2-2010/104841        US-A1- 2009 299 924
US-A1- 2015 178 469

**Beschreibung**

Die vorliegende Erfindung betrifft das technische Gebiet der Unterstützung von Patienten bei der wiederholten Einnahme von Arzneimitteln im Rahmen einer therapeutischen Behandlung. Gegenstand der vorliegenden Erfindung sind ein Verfahren, ein System und ein Computerprogrammprodukt, die die Einnahme von Arzneimitteln durch einen Patienten überwachen, und den Patienten oder einen Arzt oder eine andere Person informieren, wenn die Einnahme einer Arzneimittelportion unterbleiben sollte, um so die Effektivität der Behandlung zu optimieren oder Risiken zu reduzieren.

Es gibt eine Vielzahl an Menschen, die über einen längeren Zeitraum oder dauerhaft Arzneimittel einnehmen müssen.

Beispielsweise sollten Patienten mit Herzerkrankungen (z.B. bei künstlichem Herzklappenersatz, Herzfehlbildungen, Herzrhythmusstörungen, Herzinsuffizienz) als auch solche mit wiederholten Thrombosen und Lungenembolien langfristig eine gerinnungshemmende Therapie, umgangssprachlich auch "Blutverdünnung" genannt, erhalten.

Gerade bei einer derartigen Langzeiteinnahme von Medikamenten spielt das Thema Adhärenz eine wichtige Rolle. Adhärenz bezeichnet das Ausmaß, zu dem das Verhalten einer Person hinsichtlich Medikamenteneinnahme, Diätbefolgung und/oder Lebensstiländerungen mit den vereinbarten Empfehlungen eines medizinischen Behandlers übereinstimmt.

Es gibt verschiedene Methoden zur Adhärenzbestimmung, die in klinischen Studien sowie zur Therapiekontrolle eingesetzt werden. Sie lassen sich in direkte und indirekte Methoden unterscheiden. Beispiele für direkte Methoden sind: die direkte Beobachtung (der Einnahmen z.B. durch einen Arzt), die Messung der Arzneistoffkonzentration in Körperflüssigkeiten und die Messung von Markersubstanzen im Blut, Urin oder der Ausatmungsluft. Beispiele für indirekte Methoden sind: die Arzneimittelschwundmessung ("pill count"), Patiententagebücher und elektronisches Monitoring.

Die Patientenbefragung ist die gängigste Methode zur Adhärenzbestimmung. Jedoch scheinen Patienten ihre Adhärenz zu überschätzen, und die Angaben für längere Zeiträume werden schnell ungenau. Genauere Werte lassen sich durch Medikamentenbestimmung im Blut erreichen. Diese Methode ist jedoch kostenintensiv und gibt lediglich Aufschluss über den Zeitraum, in der die eingenommene Substanz noch nicht ausgeschieden oder verstoffwechselt ist.

Geräte zum elektronischen Monitoring werden auch unter dem Begriff MEMS (Medication Event Monitoring System(s)) zusammenfasst. Es gibt verschiedene auf dem Markt befindliche MEMS. Beispielhaft seien die "Helping Hand" der Firma Bang & Olufsen Medicom und die Smartphone-App der Firma AiCure zur visuellen Bestätigung der Einnahme einer Arzneimittelportion mittels der im Smartphone eingebauten Kamera genannt.

Die auf dem Markt befindlichen Systeme unterstützen einen Patienten bei einer, der jeweiligen Therapie entsprechenden, wiederholten Einnahme von Arzneimittelportionen.

Es können aber Ereignisse auftreten, bei denen ansonsten wiederholt eingenommene Arzneimittel nicht eingenommen werden sollten, die weitere Einnahme den Patienten sogar vital gefährden könnte. Als Beispiel seien operative oder vergleichbare interventionelle Eingriffe (z.B. Biopsien, Punktionen und dergleichen) bei solchen Patienten angeführt, die mit Antikoagulanzien oder Thrombozytenfunktionshemmern behandelt werden. Die Gefahr einer erhöhten Blutungsneigung muss hierbei nicht nur im Hinblick auf die operative Intervention, sondern auch auf die Wahl des anästhesiologischen Verfahrens (z.B. rückenmarknahes Regionalverfahren) beachtet werden.

US2013035951 besagt, dass gerinnungshemmende Medikamente eine Woche vor der Operation vermieden werden sollten, und offenbart eine Methode und ein System gemäß der Präambel von Anspruch 1 oder Anspruch 9.

Es besteht damit ein Bedarf, Patienten nicht nur an die regelmäßige Einnahme von Arzneimittelportionen zu erinnern, sondern sie auch im Falle eines geplanten Ereignisses, für das die Einnahme einer Arzneimittelportion einer effektiven Therapie entgegensteht oder ein Risiko darstellt, zu unterstützen.

Diese technische Aufgabe wird durch die Gegenstände der unabhängigen Ansprüche 1, 10 und 15 gelöst. Bevorzugte Ausführungsformen finden sich in den abhängigen Ansprüchen sowie in der vorliegenden Beschreibung.

Ein erster Gegenstand der vorliegenden Erfindung ist somit ein Verfahren umfassend die Schritte

- automatisches Überwachen der wiederholten Einnahme eines Arzneimittels durch einen Patienten
- Registrieren eines Ereignisses, für das die Einnahme des Arzneimittels kontraindiziert ist
- Ermitteln eines Absetzzeitpkts, nach dem das Arzneimittel aufgrund der Kontraindikation nicht mehr eingenommen werden sollte
- Registrieren einer versuchten oder erfolgten Einnahme des Arzneimittels nach dem Absetzzeitpunkt
- im Falle der versuchten Einnahme: Übermitteln einer Warnung an den Patienten, dass das Arzneimittel nicht eingenommen werden sollte
- im Falle einer erfolgten Einnahme: Übermitteln einer Mitteilung an den Patienten und/oder eine mit dem Ereignis in Beziehung stehende weitere Person über die erfolgte Einnahme.

Ein weiterer Gegenstand der vorliegenden Erfindung ist ein System umfassend

- Mittel zum automatischen Überwachen der wiederholten Einnahme eines Arzneimittels durch einen Patienten
- Mittel zum Registrieren eines Ereignisses, für das die Einnahme des Arzneimittels kontraindiziert ist
- Mittel zum Ermitteln eines Absetzzeitpunkts, nach dem das Arzneimittel aufgrund der Kontraindikation nicht mehr eingenommen werden sollte
- Mittel zum Registrieren einer versuchten oder erfolgten Einnahme des Arzneimittels nach dem Absetzzeitpunkt
- Mittel zum Übermitteln einer Warnung an den Patienten, dass das Arzneimittel nicht eingenommen werden sollte und/oder zum Übermitteln einer Mitteilung an den Patienten und/oder eine mit dem Ereignis in Beziehung stehende weitere Person über die erfolgte Einnahme.

Ein weiterer Gegenstand der vorliegenden Erfindung ist ein Computerprogrammprodukt umfassend einen Datenträger und Programmcode, der auf dem Datenträger gespeichert ist, und eine Computereinheit, in dessen Arbeitsspeicher der Programmcode geladen ist, dazu veranlasst, die folgenden Schritte auszuführen:

- Empfangen eines Ereignis-Zeitpunkts für ein Ereignis, für das die Einnahme eines Arzneimittels kontraindiziert ist
- Ermitteln eines Absetzzeitpunkts, ab dem das Arzneimittel aufgrund der Kontraindikation nicht mehr eingenommen werden sollte
- Registrieren eines Einnahmeversuchs oder eine erfolgten Einnahme einer Portion des Arzneimittels durch einen Patienten
- Übermitteln einer Warnung an den Patienten, dass die Arzneimittelportion nicht eingenommen werden sollte, und/oder an eine mit dem Ereignis in Beziehung stehende weitere Person über die erfolgte Einnahme.

Die Erfindung wird nachstehend näher erläutert, ohne zwischen den Erfindungsgegenständen (Verfahren, System, Computerprogrammprodukt) zu unterscheiden. Die nachfolgenden Erläuterungen sollen vielmehr für alle Erfindungsgegenstände in analoger Weise gelten, unabhängig davon, in welchem Kontext (Verfahren, System, Computerprogrammprodukt) sie erfolgen.

Wenn in der vorliegenden Beschreibung oder in den Patentansprüchen Schritte in einer Reihenfolge genannt werden, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden können; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen des erfindungsgemäßen Verfahrens dar.

Die Erfindung ist vorwiegend für Patienten gedacht, die wiederholt Arzneimittel einnehmen. Unter "wiederholter Einnahme" ist zu verstehen, dass der Patient im Rahmen einer Therapie oder einer vorbeugenden Maßnahme ein Arzneimittel über einen Zeitraum, der sich üblicherweise über mehrere Tage, Wochen, Monate oder sogar Jahre erstreckt, einnehmen muss. Man könnte sagen, dass eine wiederholte Einnahme im Sinne der vorliegenden Erfindung dann vorliegt, wenn der Patient mehr als fünf, vorzugsweise mehr als zehn, besonders bevorzugt mehr als zwanzig Arzneimittelportionen eines spezifischen Arzneimittels zu unterschiedlichen Zeitpunkten einnehmen muss. Die Einnahme der Arzneimittelportionen kann regelmäßig oder unregelmäßig erfolgen. Üblicherweise erfolgt die Einnahme regelmäßig, das bedeutet in zeitlich konstanten Abständen.

Unter einem Arzneimittel wird eine Substanz oder ein Substanzgemisch bezeichnet, das eine therapeutische Wirkung ausübt. Ein zum Begriff Arzneimittel synonymer Begriff ist der Begriff Medikament.

Der Begriff "Einnahme" soll nicht einschränkend dahingehend verstanden werden, dass er nur eine orale Verabreichung eines Arzneimittels bedeutet. Vielmehr soll jede denkbare Applikationsform unter den Begriff "Einnahme" fallen, wie z.B. aural, buccal, inhalativ, intraarteriell, intraartikulär, intraglutäal, intrakutan, intramuskulär, intraokular, intrauterin, intravenös, intravitreal, intranasal, perkutan, rektal, sublingual, subkutan, topisch, transdermal, vaginal und dergleichen. Erfindungsgemäß wird das Arzneimittel durch den Patienten selbst eingenommen; d.h. eine ärztliche Fachkraft ist nicht erforderlich, um das Arzneimittel dem Patienten zu verabreichen. Vorzugsweise handelt es sich um ein Arzneimittel, das der Patient oral einnimmt.

Üblicherweise liegt das Arzneimittel in Form von definierten Portionen vor, von denen ein Patient eine definierte Menge (eine Portion, zwei Portionen, eine halbe Portion oder dergleichen) zu definierten Zeitpunkten oder innerhalb definierter Zeitspannen einnehmen soll.

Das Arzneimittel kann fest (z.B. in Form von Tabletten) oder flüssig (z.B. als Saft) oder gasförmig oder in einer Mischform (z.B. als Gelkapsel oder als Aerosol oder als Salbe) vorliegen. Es kann sich um einen Reinstoff, eine Feststoffmischung, eine Lösung, eine Suspension (z.B. eine Emulsion oder ein Aerosol) oder dergleichen handeln.

In einer besonders bevorzugten Ausführungsform handelt es sich bei dem Arzneimittel um ein Antikoagulans oder ein Thrombozytenfunktionshemmer.

Beispiele für Antikoagulanzien sind z.B. Rivaroxaban, Dabigatran, Apixaban, Edoxaban, Cumarine (z.B. Marcumar, Warfarin), Heparine (wie z.B. Dalteparin, Enoxaparin, Tinzaparin, Fondaparinux, u.a.), Faktor XIa und Faktor XI Inhibitoren.

Beispiele für Thrombozytenfunktionshemmer sind Aspirin, Clopidogrel, Prasugrel, Ticagrelor, Cangrelor, Elinogrel, Abciximab, Eptifibatid, Tirofiban, Vorapaxar, Atopaxar, Tifacogin, NAPc2.

Ein erster Schritt des erfindungsgemäßen Verfahrens ist das automatische Überwachen der wiederholten Einnahme eines Arzneimittels durch einen Patienten. Dabei bedeutet der Begriff "Überwachen der Einnahme", dass kontrolliert wird, ob der Patient zumindest Vorkehrungen unternommen hat, um eine Arzneimittelportion einzunehmen, und/oder ob er tatsächlich eine Arzneimittelportion eingenommen hat.

Die Überwachung der Einnahme geschieht, um die Adhärenz zu erhöhen. "Adhärenz" bezeichnet hier das Ausmaß, in dem das Verhalten des Patienten in Bezug auf die Einnahme eines Arzneimittels mit den mit dem Therapeuten vereinbarten Empfehlungen übereinstimmt.

Der Begriff "automatisches Überwachen" bedeutet, dass der Patient bei der wiederholten Einnahme eines Arzneimittels durch eine technische Überwachungseinheit für eine medikamentöse Behandlung (kurz: MEMS = Medication Event Monitoring System) unterstützt wird. Die Überwachungseinheit ist ein Bestandteil des erfindungsgemäßen Systems. Die Überwachungseinheit ist vorzugsweise ein transportables Gerät, das ein Patient mit sich führen kann (z.B. in der Hosentasche oder der Handtasche).

Unter dem Begriff Überwachungseinheit wird jedes elektronische System verstanden, das einen Einnahmeversuch und/oder eine tatsächliche Einnahme einer Arzneimittelportion durch eine Person erkennt.

Unter dem Begriff "Einnahmeversuch" werden Maßnahmen verstanden, die von einer Person unternommen werden, um eine Einnahme einer Arzneimittelportion vorzubereiten. Ein typisches Beispiel ist die Entnahme einer Arzneimittelportion aus einer Verpackung, z.B. die Entnahme einer Tablette aus einer Blisterverpackung.

Es ist denkbar, in Ausführungsformen, die nicht der Erfindung entsprechen, dass vorbereitende Maßnahmen zur Einnahme einer Arzneimittelportion nicht durch den Patienten selbst sondern z.B. durch einen Arzt oder durch Pflegepersonal oder durch Angehörige vorgenommen werden.

In einer Ausführungsform der vorliegenden Erfindung registriert die Überwachungseinheit, ob und wann der Patient Vorbereitungen zur Entnahme einer Arzneimittelportion aus einer Vorrichtung zur Aufbewahrung des Arzneimittels getroffen hat und/oder ob und wann der Patient eine Arzneimittelportion entnommen hat. Es ist denkbar, dass der Patient, bevor er der Aufbewahrungsvorrichtung eine Arzneimittelportion entnehmen kann, zum Bespiel durch Drücken einer Taste oder durch Präsentieren eines biometrischen Merkmals (zum Beispiel des Fingers zur Erfassung eines Fingerabdrucks im Rahmen einer Fingerabdruckerkennung) signalisieren muss, dass er eine Arzneimittelportion entnehmen möchte. In einem solchen Fall registriert die Überwachungseinheit die Aktion des Patienten, die in einer Entnahme einer Arzneimittelportion resultieren soll. Es ist aber auch denkbar, dass die Überwachungseinheit die tatsächliche Entnahme einer Arzneimittelportion registriert. Es ist zum Beispiel denkbar, dass durch Herausdrücken einer Arzneimittelportion aus einer Blisterverpackung eine elektrisch leitende Leiterbahn unterbrochen wird; diese Unterbrechung kann zum Beispiel durch eine elektronische Schaltung erfasst werden (siehe z.B. WO9604881A1 oder DE19516076A1).

In einer anderen Ausführungsform ist die Überwachungseinheit so ausgeführt, dass sie die tatsächliche Einnahme einer Arzneimittelportion durch den Patienten registriert. Beispielhaft sei hier das bereits oben erwähnte System der Firma AiCure erwähnt. Im Fall des AiCure-Systems wird die tatsächliche Einnahme einer Arzneimittelportion von einer Smartphone-App mit Hilfe der Kamera des Smartphones verfolgt. Bildanalyse- und Bilderkennungsalgorithmen sorgen dafür, dass die Arzneimittelportion und das Gesicht des Patienten erkannt werden. Ferner wird erkannt, dass der Patient die Arzneimittelportion in den Mund steckt und schluckt.

In einer bevorzugten Ausführungsform ist die Überwachungseinheit so ausgeführt, dass sie den Patienten (oder auch das Pflegepersonal oder eine andere Person) an eine anstehende Einnahme einer Arzneimittelportion erinnert, z.B. akustisch (z.B. mittels eines Signaltons oder einer Sprachnachricht), visuell (z.B. durch ein Blinken eines Lämpchens oder einer Textnachricht) und/oder taktil (z.B. mittels Vibration).

In einem weiteren Schritt des erfindungsgemäßen Verfahrens wird ein Ereignis registriert. Das Ereignis ist für die Einnahme des Arzneimittels kontraindiziert. Das bedeutet, dass Gründe vorliegen, die gegen die Einnahme des Arzneimittels zu einem Zeitpunkt sprechen, bei dem das Arzneimittel eine Wirkung entfaltet, die bei dem Ereignis noch anhält.

Es ist denkbar, dass sich eine Einnahme einer Arzneimittelportion nicht mit dem Ereignis "verträgt". Die Wirkung des Arzneimittels führt zu einem Effekt, "der nicht zu dem Ereignis passt" oder im Zusammenhang mit dem Ereignis "unerwünscht ist".

Der Patient ist also im Normalfall angehalten, ein Arzneimittel wiederholt einzunehmen; aufgrund des Ereignisses sollte er jedoch von einer (weiteren) Einnahme absehen.

In einer bevorzugten Ausführungsform handelt es sich bei dem Ereignis um ein für die Zukunft geplantes Ereignis.

Vorzugsweise handelt es sich bei dem Ereignis um eine medizinische Intervention, besonders bevorzugt um eine Maßnahme, die mit Blutungen einhergehen oder Blutungen verursachen kann.

Als Intervention bezeichnet man in der Medizin jede aktive Form von Behandlung, wenn man sie von einem bloßen Zuwarten unterscheiden möchte. Dies umfasst therapeutische und präventive Maßnahmen gleichermaßen. Im engeren Sinne bedeutet Intervention ein akutes, dringliches Einschreiten gegen einen Krankheitsprozess.

Beispiele für Interventionen, die mit Blutungen verbunden sein können, sind chirurgische Eingriffe (Operationen), Punktionen, Biopsien, Zahnextraktionen, Katheter-Untersuchungen und dergleichen.

Es ist zum Beispiel denkbar, dass die Wirkung des Arzneimittels für das Ereignis ein (erhöhtes) Risiko (z.B. ein Sicherheitsrisiko) darstellt. Es ist zum Beispiel denkbar, dass das Arzneimittel eine "blutverdünnende" Wirkung ausübt und es sich bei dem für die Zukunft geplanten Ereignis um eine invasive Maßnahme handelt. Durch die "blutverdünnende" Wirkung des Arzneimittels besteht ein erhöhtes Risiko von Blutungen, die während der invasiven Maßnahme und/oder danach auftreten und nicht oder nur schwer gestoppt werden können.

Es ist aber auch denkbar, dass ein Arzneimittel regelmäßig abgesetzt werden muss. Als Beispiel seien Kontrazeptiva aufgeführt, die üblicherweise über einen Turnus von 21 Tagen eingenommen werden; danach wird die Einnahme für einen Zeitraum von 7 Tagen unterbrochen. Die Patientin sollte also 21 Tage lang eine Arzneimittelportion einnehmen und wird vorzugsweise auch daran erinnert. Versucht sie aber am 22. Tag eine weitere Arzneimittelportion einzunehmen, wird dieser Versuch erfindungsgemäß erkannt und es werden entsprechende Maßnahmen ergriffen.

Es ist ferner denkbar, dass es sich bei dem Ereignis um das Erreichen eines Wirkspiegels bei einem Patienten handelt. Unter einem "Wirkspiegel" wird die Konzentration eines Arzneimittels im Körper (oder bestimmten Körperregionen) eines Patienten verstanden, die erforderlich ist, um eine gewünschte Wirkung des Arzneimittels zu erzielen. Es ist denkbar, dass durch eine wiederholte Einnahme eines Arzneimittels ein Wirkspiegel erreicht wird. Es ist denkbar, dass das Arzneimittel nach Erreichen des Wirkspiegels abgesetzt werden kann oder abgesetzt werden sollte, um Nebenwirkungen zu verringern oder zu vermeiden. Ein solches Vorgehen könnte beispielsweise bei einer Antikörpertherapie sinnvoll sein.

Denkbar ist aber auch, dass sich das Ereignis dadurch ergibt, dass ein Parameter im Körper des Patienten einen definierten Wert annimmt oder einen Wert ober- oder unterhalb eines Schwellenwerts erreicht. Der Parameter kann z.B. die Konzentration eines Arzneimittels im Körper oder in einer Körperregion sein; z.B. kann es die Konzentration eines Arzneimittels im Blut oder einer anderen Körperflüssigkeit sein. Der Parameter kann auch die Konzentration eines Biomarkers oder einer anderen im Körper oder in Ausscheidungsprodukten des Körpers messbaren Substanz sein. Bei dem Parameter kann es sich auch um die Körpertemperatur, den Blutdruck oder andere physiologische Größen handeln. Vorzugsweise wird der Parameter mit Hilfe eines Sensors am oder im Körper eines Patienten oder in einem Körperprodukt des Menschen (Blut, Speichel, Urin usw.) gemessen.

In einem weiteren Schritt des erfindungsgemäßen Verfahrens wird ein Absetzzeitpunkt ermittelt. Der Absetzzeitpunkt liegt in einer bevorzugten Ausführungsform der vorliegenden Erfindung zeitlich vor dem (vorzugsweise für die Zukunft geplanten) Ereignis.

Bekanntermaßen entfaltet ein Arzneimittel seine Wirkung über einen gewissen Zeitraum. Nach einer Einnahme des Arzneimittels hält die Wirkung des Arzneimittels für einen gewissen Zeitraum an, der üblicherweise patientenspezifisch ist.

Der Absetzzeitpunkt ist so definiert, dass nach diesem Zeitpunkt das Arzneimittel nicht mehr eingenommen werden sollte, um eine (unerwünschte) Wirkung des Arzneimittels zum Zeitpunkt des Ereignisses zu verhindern oder dafür Sorge zu tragen, dass die Wirkung unterhalb einer Toleranzschwelle liegt.

Es ist denkbar, den Absetzzeitpunkt mit Hilfe von Wirkzeitspannen zu berechnen. Dabei können beispielsweise mittlere Wirkzeitspannen oder sichere Wirkzeitspannen verwendet werden.

Eine Wirkzeitspanne beginnt üblicherweise mit der Einnahme des Arzneimittels. Die mittlere Wirkzeitspanne endet, wenn im Mittel bei Patienten keine Wirkung des Arzneimittels mehr zu beobachten/zu messen ist. Die sichere Wirkzeitspanne endet zu dem Zeitpunkt, zu dem mit an Sicherheit grenzender Wahrscheinlichkeit davon ausgegangen werden kann, dass das Arzneimittel keine Wirkung mehr entfaltet. Es ist aber auch denkbar, andere Wirkzeitspannen zu definieren; z.B. ist es denkbar, als Wirkzeitspanne die Zeitspanne zu definieren, bei der die Konzentration des Arzneimittels auf einen bestimmten Betrag z.B. des Maximalwerts gesunken ist (z.B. 1% oder 5% oder 10% oder dergleichen). Es geht also bei der Definition der Wirkzeitspanne darum, zunächst diejenige Wirkung eines Arzneimittels zu definieren, die bei dem (vorzugsweise für die Zukunft geplanten) Ereignis noch tolerierbar ist. Anschließend wird die Zeit gemessen, die vergeht, bis das Arzneimittel nach Einnahme durch den Patienten die tolerierbare Wirkung erreicht hat.

Wirkzeitspannen können beispielsweise in klinischen Studien ermittelt werden.

Vorzugsweise wird der Absetzzeitpunkt dadurch errechnet, dass von dem Ereignis-Zeitpunkt die Wirkzeitspanne abgezogen wird:

$$\text{Absetzzeitpunkt} = \text{Zeitpunkt des für die Zukunft geplanten Ereignisses} - \text{Wirkzeitspanne}$$

In einem solchen Fall würde die Wirkung eines Arzneimittels, das zum Absetzzeitpunkt genommen werden würde, innerhalb der Wirkzeitspanne abklingen und hätte zum Ereignis-Zeitpunkt die noch tolerierbare Wirkung. Ein Arzneimittel, das nach dem Absetzzeitpunkt genommen werden würde, hätte zum Ereignis-Zeitpunkt noch eine Wirkung, die oberhalb der Toleranzschwelle liegt.

Wirkzeitspannen können in Datenbanken gespeichert sein. Es ist denkbar, dass das erfindungsgemäße System auf eine solche Datenbank zugreifen kann, um die Wirkzeitspanne für das jeweilige Arzneimittel zu ermitteln. Es ist denkbar, dass das erfindungsgemäße System über eine Speichereinheit verfügt, in der die Wirkzeitspanne gespeichert ist.

In einer bevorzugten Ausführungsform wird die Wirkzeitspanne für den jeweiligen Patienten auf Basis von patientenspezifischen Informationen errechnet. Die Berechnung kann zu dem Zeitpunkt erfolgen, an dem das Registrieren des für die Zukunft geplanten Ereignisses stattfindet; sie kann aber auch früher oder später erfolgen. Die Berechnung kann mit Hilfe des erfindungsgemäßen Systems erfolgen.

Patientenspezifische Informationen, die in die Berechnung der Wirkzeitspanne einfließen können, sind beispielsweise Geschlecht, Alter, Gewicht, Größe, Blutwerte, genetische Merkmale u.v.m.

Es ist denkbar, für die Berechnung der patientenspezifischen Wirkzeitspanne ein pharmakokinetisches Metabolismus-Model, wie man es beispielsweise mit PKSim© (http://www.systems-biology.com/products/pk-sim.html) erstellen kann, zu verwenden.

Denkbar ist aber auch, dass der Absetzzeitpunkt mit dem Ereignis-Zeitpunkt zeitlich zusammenfällt. Es ist zum Beispiel denkbar, dass ein Parameter beim Patienten einen definierten Wert erreicht oder einen Wert ober- oder unterhalb eines Schwellenwertes annimmt. Das kann das auslösende Ereignis für das (zumindest zeitweise) Absetzen eines Arzneimittels sein. Nach diesem Ereignis-Zeitpunkt (=Absetzzeitpunkt), sollte das Arzneimittel also nicht mehr eingenommen werden.

In einer Ausführungsform der vorliegenden Erfindung registriert das erfindungsgemäße System den Versuch der Einnahme einer Arzneimittelportion nach dem Absetzzeitpunkt. Eine Person (üblicherweise der Patient, aber auch ein Arzt oder Pflegepersonal oder eine andere Person) ergreift eine Maßnahme, die üblicherweise zu einer Einnahme einer Arzneimittelportion durch den Patienten führt. Die Person kann beispielsweise eine Arzneimittelportion aus einem Behältnis entnehmen, wobei diese Entnahme registriert wird. Denkbar ist aber auch, dass die Person zur Entnahme einer Arzneimittelportion aus einem Behältnis einen Schalter betätigen oder ein biometrisches Merkmal präsentieren muss. In einem solchen Fall wird als Entnahmeversuch das Betätigen des Schalters oder die Präsentation eines biometrischen Merkmals registriert. Bei dem Schalter kann es sich auch um einen virtuellen Schalter auf einem Bildschirm einer Computereinheit handeln.

Erfindungsgemäß erfolgt in Reaktion auf den Einnahmeversuch eine Warnung, um die tatsächliche Einnahme zu verhindern. Die Warnung erfolgt üblicherweise gegenüber derjenigen Person, die den Einnahmeversuch unternommen hat. Es ist aber auch denkbar, eine oder mehrere Warnungen gegenüber einer anderen oder einer oder mehreren weiteren Personen abzugeben.

Eine solche Warnung kann akustisch (z.B. mittels eines Signaltons oder einer Sprachnachricht), visuell (z.B. mittels eines aufblickenden Lämpchens oder einer Textnachricht) und/oder taktil (z.B. mittels Vibration) erfolgen.

Es ist auch denkbar, dass einem Arzt oder Pflegepersonal eine elektronische Nachricht (E-Mail, SMS oder dergleichen) zugesandt wird, die auf den Einnahmeversuch hinweist.

In einer Ausführungsform der vorliegenden Erfindung registriert das erfindungsgemäße System die tatsächliche Einnahme einer Arzneimittelportion nach dem Absetzzeitpunkt. Erfindungsgemäß erfolgt als Reaktion auf die erfolgte Einnahme die Übermittlung einer Mitteilung. Die Mitteilung kann sich an den Patienten, einen Arzt und/oder das Pflegepersonal und/oder an eine andere Person richten, die einen Bezug zu dem für die Zukunft geplanten Ereignis hat, richten. Die Mitteilung kann darauf hinweisen, dass eine Arzneimittelportion nach dem Absetzzeitpunkt vom Patienten eingenommen worden ist. Als Reaktion auf die erfolgte Einnahme kann das für die Zukunft geplante Ereignis abgesagt oder verschoben werden. Es ist auch denkbar, dass eine Absage oder Verschiebung nicht möglich oder nicht gewollt ist. Die Mitteilung kann dann dazu verwendet werden, um (andere) Maßnahmen zu ergreifen, die durch die (unerwünschte) Wirkung des Arzneimittels zum Zeitpunkt des geplanten zukünftigen Ereignisses verursachten Effekte zu verhindern oder zu reduzieren oder zu kompensieren.

Bei dem erfindungsgemäßen System kann aus sich um eine einzige Vorrichtung (ein einziges Gerät) handeln; denkbar ist aber auch, dass die Funktionen, die das erfindungsgemäße System ausübt, auf zwei oder mehr Vorrichtungen (Geräte) verteilt sind.

In einer Ausführungsform liegt eine einzige Vorrichtung vor. In der erfindungsgemäßen Vorrichtung sind die verschiedenen Funktionalitäten, die zur Realisierung der vorliegenden Erfindung erforderlich sind, in einem einzigen Gerät zusammengefasst. Die Vorrichtung weist Mittel zur Aufnahme mehrerer Arzneimittelportionen auf. Die Vorrichtung kann z.B. ein Behältnis umfassen, in das mehrere Arzneimittelportionen eingefüllt werden können. Denkbar ist aber auch, dass die Vorrichtung ein auswechselbares Behältnis für Arzneimittelportionen (z.B. einen Blisterstreifen) aufnehmen kann. Die erfindungsgemäße Vorrichtung kann Mittel aufweisen, die einen Entnahmeversuch oder eine tatsächliche Entnahme einer Arzneimittelportion registrieren. Alternativ kann die erfindungsgemäße Vorrichtung Mittel aufweisen, die eine tatsächliche Einnahme einer Arzneimittelportion durch einen Patienten registriert. Die Vorrichtung umfasst eine Computereinheit, die so konfiguriert ist, dass sie ein Ereignis registrieren kann, d.h. das Datum und ggf. die Uhrzeit eines Ereignisses wird festgehalten. Denkbar ist z.B., dass die Vorrichtung eine Kalenderfunktion umfasst, in der ein Zeitpunkt für ein Ereignis eingetragen werden kann. Denkbar ist aber auch, dass die Vorrichtung in einer kommunikativen Verbindung mit einem Sensor steht. Denkbar ist, dass der Sensor einen Parameter im oder am Körper eines Patienten misst und Messwerte an die erfindungsgemäße Vorrichtung übermittelt. Sobald ein übermittelter Messwert einen definierten Wert erreicht hat oder einen Wert annimmt, der ober- oder unterhalb eines definierten Schwellenwerts liegt, registriert die Computereinheit das entsprechende Ereignis. Zur Registrierung eines Ereignisses zählen üblicherweise der Zeitpunkt des Ereignisses (Ereignis-Zeitpunkt) sowie Informationen darüber, um was für ein Ereignis es sich handelt. Die Computereinheit ist ferner so konfiguriert, dass sie anhand des Ereignisses einen Absetzzeitpunkt ermitteln, vorzugsweise berechnen kann. Unternimmt eine Person einen Einnahmeversuch oder eine tatsächliche Einnahme, wird von der Computereinheit ermittelt, ob der Einnahmeversuch oder die tatsächliche Einnahme zeitlich vor oder nach dem Absetzzeitpunkt erfolgt. Erfolgt der Einnahmeversuch oder die tatsächliche Einnahme zeitlich nach dem Absetzzeitpunkt, übermittelt die Computereinheit eine Mitteilung an die Person, die den Einnahmeversuch oder die tatsächliche Einnahme unternommen hat. Dazu weist die erfindungsgemäße Vorrichtung entsprechende Mittel auf, wie beispielsweise einen Bildschirm (für eine Text- oder Bildnachricht), ein Lämpchen (für ein optisches Warnsignal), einen Lautsprecher (für eine Sprachnachricht oder einen Warnton) und/oder einen Vibrationsalarm. Ferner kann die Computereinheit so konfiguriert sein, dass sie eine Mitteilung an eine andere oder weitere Person übermittelt, z.B. eine E-Mail oder eine SMS oder dergleichen an einen Arzt und/oder an Pflegepersonal oder dergleichen.

Es ist auch denkbar, dass verschiedene Funktionalitäten, die zur Realisierung der vorliegenden Erfindung erforderlich sind, auf mehrere separate Geräte verteilt sind. Es ist zum Beispiel denkbar, dass es eine separate Überwachungseinheit gibt, die eine kommunikative Verbindung zu einer separaten Computereinheit aufnehmen kann. Es ist zum Beispiel denkbar, dass die Überwachungseinheit so konfiguriert ist, dass sie Informationen zu einem Einnahmeversuch oder zu einer tatsächlichen Einnahme an die separate Computereinheit (z.B. über Bluetooth, ZigBee, ein Mobilfunk-Netzwerk oder dergleichen) übermitteln kann. Dabei kann die Überwachungseinheit so konfiguriert sein, dass sie jeglichen Einnahmeversuch oder jegliche Einnahme übermittelt; es ist aber auch denkbar, dass sie nur Einnahmeversuche oder Einnahmen nach dem Absetzzeitpunkt übermittelt. Die separate Computereinheit, die vorzugsweise als Smartphone, Smartwatch oder Tablet-Computer ausgeführt ist, ist so konfiguriert, dass sie Informationen zum Einnahmeversuch oder zur tatsächlichen Einnahme von der Überwachungseinheit empfangen und eine oder mehrere Mitteilungen abgeben kann.

Ferner ist es denkbar, dass die erfindungsgemäße Vorrichtung oder die Überwachungseinheit eine Sperrfunktion aufweist, die aktiviert wird, sobald ein Einnahmeversuch nach dem Absetzzeitpunkt registriert wird. Die Sperrfunktion verhindert beispielsweise eine Entnahme einer Arzneimittelportion, indem sie beispielsweise den Zugang zu dem Behältnis, in dem sich Arzneimittelportionen befinden, blockiert.

Unter dem Begriff Computereinheit oder auch kurz Computer wird vorzugsweise ein universell programmgesteuerter Automat zur Informationsverarbeitung verstanden. Eine Computereinheit verfügt über mindestens eine Eingabeeinheit, über die Daten und Steuerbefehle eingegeben werden können (Maus, Trackpad, Tastatur, Scanner, Webcam, Joystick, Mikrofon, Barcodeleser usw.), eine Verarbeitungseinheit umfassend Arbeitsspeicher und Prozessor, mit der Daten und Befehle verarbeitet werden, und eine Ausgabeeinheit, um Daten aus dem System zu senden (z.B. Bildschirm, Drucker, Lautsprecher usw.). Heutige Computer werden oft in Desktop-Computer, Portable Computer, Laptops, Notebooks, Netbooks und Tablet-Computer und so genannte Handhelds (z.B. Smartphones, Smartwatches) unterteilt.

Die Erfindung wird nachfolgend anhand von Figuren und bevorzugten Ausführungsformen näher erläutert, ohne die Erfindung auf diese Bespiele reduzieren zu wollen.

Es zeigen:

Fig. 1 zeigt schematisch eine Ausführungsform des erfindungsgemäßen Systems.
Das erfindungsgemäße System (1) umfasst eine Überwachungseinheit (10), Mittel (20) zur Aufnahme von Arzneimittelportionen und eine Computereinheit (30).

Fig. 2 zeigt schematisch eine weitere Ausführungsform des erfindungsgemäßen Systems.
Das erfindungsgemäße System (1) umfasst zwei separate Komponenten. Die eine Komponente ist eine Überwachungseinheit (10), die andere Komponente ist eine Computereinheit (30). Die Überwachungseinheit (10) weist einen Behälter (25) zur Aufnahme von Arzneimittelportionen und eine Sensoreinheit (15) auf. Die Sensoreinheit (15) ist so ausgestaltet, dass sie eine Entnahme und/oder einen Entnahmeversuch einer Arzneimittelportion aus dem Behälter (25) registriert und eine entsprechende Information über die Entnahme und/oder den Entnahmeversuch über eine Sendeeinheit (16) an die Überwachungseinheit (30) übermittelt. Die Überwachungseinheit (30) weist eine Empfangseinheit (36) auf, mit der sie die Information von der Sendeeinheit (16) empfangen kann.

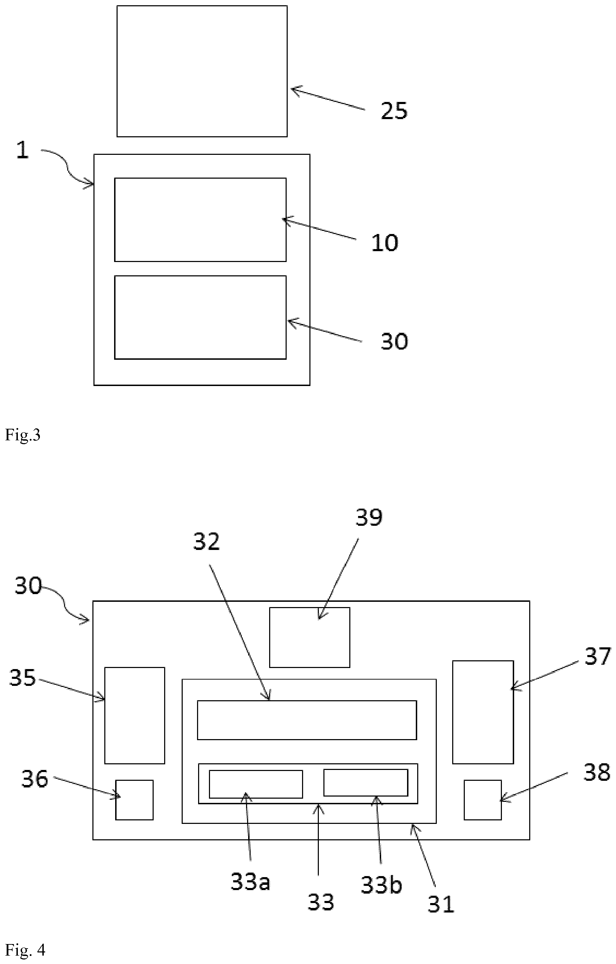

Fig. 3 zeigt schematisch eine weitere Ausführungsform des erfindungsgemäßen Systems.

Eine Überwachungseinheit (10) und eine Computereinheit (30) sind in einer einzigen (erfindungsgemäßen) Vorrichtung (1) integriert. Mit der Überwachungseinheit (10) kann eine Entnahme und/oder ein Entnahmeversuch einer Arzneimittelportion aus einem separaten Behälter (25) erfasst werden.

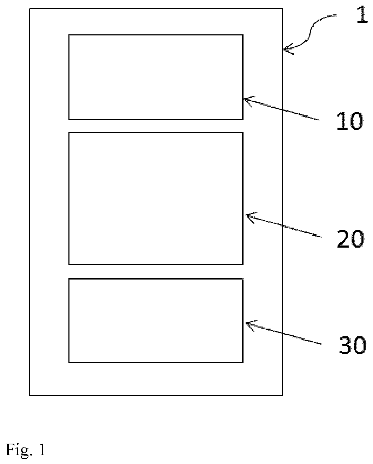

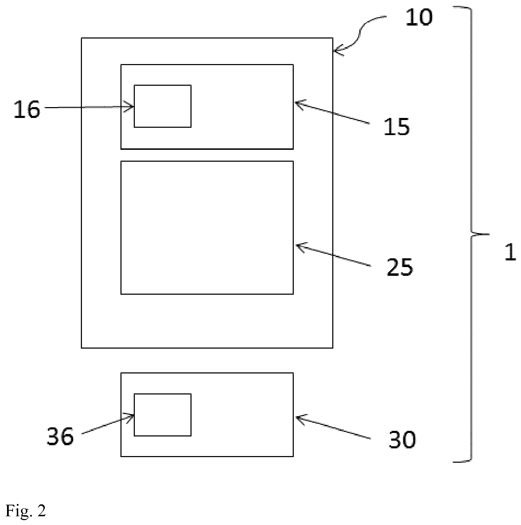

Fig. 4 zeigt schematisch ein Beispiel für den Aufbau einer Computereinheit des erfindungsgemäßen Systems, wie es beispielhaft in den Figuren 1, 2 und 3 gezeigt ist.

Die Computereinheit (30) umfasst eine Eingabeeinheit (35), mit der ein Nutzer Daten und Steuerbefehle eingeben kann. Sie umfasst eine Verarbeitungseinheit (31) zum Verarbeiten von Daten und Steuerbefehlen. Die Verarbeitungseinheit (31) weist einen Arbeitsspeicher (32) und einen Prozessor (33) auf. Der Prozessor (33) umfasst ein Steuerwerk (33a) und ein Rechenwerk (33b). Die Computereinheit (30) umfasst ferner eine Ausgabeeinheit (37), mit der Daten ausgegeben werden können. Die Computereinheit (30) umfasst ferner einen Datenspeicher (39), eine Empfangseinheit (36), mit der Signale und/oder Daten von externen Geräten empfangen werden können, und eine Sendeeinheit (39), mit der Signale und/oder Daten an externe Geräte verschickt werden können.

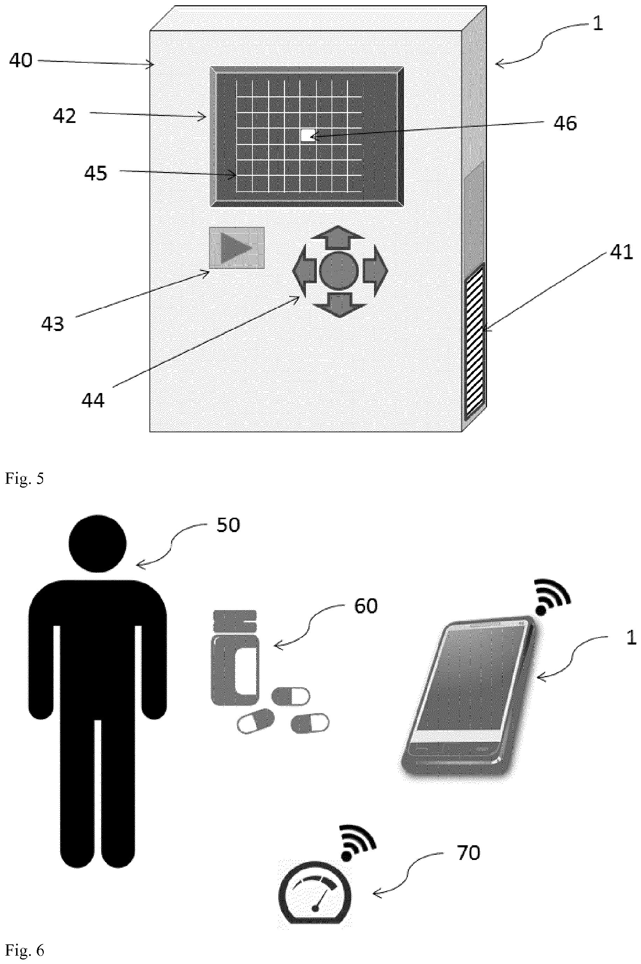

Fig. 5 zeigt ein Beispiel für ein erfindungsgemäßes System (1), in dem eine Computereinheit, eine Überwachungseinheit und ein Behälter zur Aufnahme von Arzneimittelportionen in einer einzigen Vorrichtung integriert sind. Das erfindungsgemäße System (1) ist als transportables Handheld-Gerät ausgeführt. Alle Komponenten sind in einem Gehäuse (40) untergebracht. In dem Gehäuse befindet sich ein Innenraum, in dem Arzneimittelportionen gelagert werden können (in Fig. 5 nicht explizit zu erkennen). Die Arzneimittelportionen können einzeln über eine Öffnung, die mit einem Verschluss (41) verschlossen ist, entnommen werden. Der Verschluss (41) wird zum Öffnen nach oben geschoben. Das erfindungsgemäße System verfügt über eine Ausgabeeinheit, die als Bildschirm (42) ausgeführt ist. Zur Eingabe von Daten und Steuerbefehlen dienen Tasten (43, 44) als Eingabeeinheiten. Auf dem Bildschirm (42) ist schematisch ein Kalender (45) dargestellt. In dem Kalender (45) ist ein Ereignis (46) eingetragen, für das die Einnahme des Arzneimittels kontraindiziert ist. Es kann aber auch sein, dass es sich bei dem im Kalender (45) eingetragenen Ereignis (46) um den Absetzzeitpunkt handelt, ab dem das Arzneimittel nicht mehr eingenommen werden sollte.

Folgendes ist denkbar:

- Ein Nutzer verwendet das erfindungsgemäße System (1) zur wiederkehrenden Einnahme von Arzneimittelportionen.
- Der Nutzer hat mittels der Tasten (43, 44) ein Ereignis (46) in den Kalender (45) eingetragen, für das die Einnahme des Arzneimittels kontrainduziert ist.
- Das erfindungsgemäße System ist so konfiguriert, dass es einen Absetzzeitpunkt errechnet, ab dem der Nutzer aufgrund des anstehenden Ereignisses keine Arzneimittelportion mehr einnehmen sollte.
- Das erfindungsgemäße System ist so konfiguriert, dass es einen Einnahmeversuch registriert.
- Falls der Einnahmeversuch nach dem Absetzzeitpunkt und vor dem Ereignis erfolgt, wird ein Warnhinweis auf dem Bildschirm (42) angezeigt, dass der Nutzer aufgrund des anstehenden Ereignisses keine Arzneimittelportion einnehmen sollte.

Zum Beispiel könnte der Verschluss (41) verriegelt sein und sich die Verriegelung nur durch Betätigen einer Taste, zum Beispiel der Taste (43) öffnen lassen. Falls der Nutzer die Taste (43) zum Entriegeln des Verschlusses nach dem Absetzzeitpunkt und vor dem Ereignis betätigt, könnte der Warnhinweis angezeigt werden.

Das erfindungsgemäße System könnte so konfiguriert sein, dass es den Nutzer an die wiederkehrende Einnahme einer Arzneimittelportion erinnert, so lange der Absetzzeitpunkt noch nicht erreicht ist.

Das erfindungsgemäße System könnte so konfiguriert sein, dass es eine Mitteilung an einen Arzt übermittelt, falls der Nutzer eine Arzneimittelportion nach dem Absetzzeitpunkt entnommen hat.

Fig. 6 zeigt eine weitere Ausführungsform der vorliegenden Erfindung. Ein Patient (50) muss im Rahmen einer Therapie wiederholt ein Arzneimittel (60) in Form von einzelnen Arzneimittelportionen (hier dargestellt durch die drei Kapseln) einnehmen. Das erfindungsgemäße System ist als Smartphone (1) ausgeführt, auf dem das erfindungsgemäße Computerprogramm installiert ist. Es ist denkbar, dass das Smartphone (1) so konfiguriert ist, dass es den Patienten zu definierten Zeitpunkten an die Einnahme einer Arzneimittelportion (oder mehrerer Portionen) erinnert. Das Smartphone (1) ist ferner so konfiguriert, dass es mit Hilfe eines Sensors (70) das Eintreten eines Ereignisses registriert, für das die weitere Einnahme des Arzneimittels (60) kontrainduziert ist. Dazu weist der Sensor (70) eine Sendeeinheit und das Smartphone (1) eine Empfangseinheit auf (dargestellt durch das Funksymbol). Es ist zum Beispiel denkbar, dass der Sensor einen Parameter im oder am Körper des Patienten (50) misst oder einen Parameter in einer Körperflüssigkeit (Blut, Speichel, Urin oder dergleichen) des Patienten (50) misst. Es ist denkbar, dass der Sensor (70) zu definierten Zeitpunkten gemessene Werte an das Smartphone (1) überträgt oder das Smartphone Messwerte zu definierten Zeitpunkten vom Sensor (70) abfragt. Denkbar ist auch, dass der Sensor so konfiguriert ist, dass er nur bei Über- oder Unterschreiten von definierten Grenzwerten Daten an das Smartphone (1) übermittelt. Es ist denkbar, dass das Smartphone (1) so konfiguriert ist, dass es die vom Sensor (70) empfangenen Daten mit Referenzdaten, die im Smartphone (1) in einer Speichereinheit hinterlegt (gespeichert) sind, vergleicht und bei Erreichen definierter Zustände einen Warnhinweis an den Patienten (50) und/oder an eine andere Person wie zum Beispiel einen Arzt übermittelt. Der Warnhinweis soll dann dazu dienen, den Patienten (50) von einer weiteren Einnahme des Arzneimittels (60) abzuhalten.

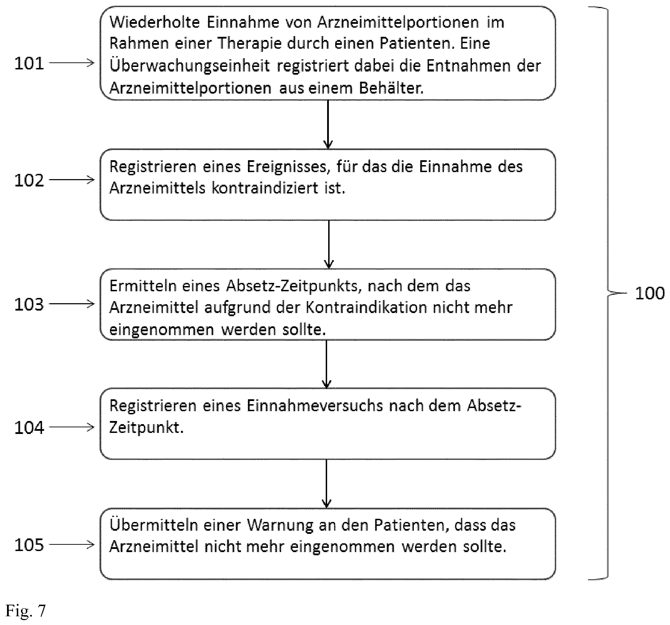

Figur 7 zeigt beispielhaft eine Ausführungsform des erfindungsgemäßen Verfahrens (100), umfassend die Schritte (101), (102), (103), (104) und (105). Ein erster Schritt (101) symbolisiert die wiederholte Einnahme von Arzneimittelportionen durch einen Patienten im Rahmen einer Therapie. Der Patient wird dabei durch eine Überwachungseinheit unterstützt; die Überwachungseinheit registriert die Entnahmen der Arzneimittelportionen aus ihrem Behälter. In einem weiteren Schritt (102) wird ein Ereignis registriert, für das die weitere Einnahme des Arzneimittels kontraindiziert ist. Denkbar ist zum Beispiel, dass es sich bei dem Arzneimittel um ein Antikoagulans oder um einen Thrombozytenfunktionshemmer handelt und der Patient die Mitteilung über eine anstehende Intervention erhält, die mit Blutungen verbunden sein kann. Es ist denkbar, dass der Patient das Ereignis in einem virtuellen Kalender, der Bestandteil der Überwachungseinheit oder einer Computereinheit sein kann, einträgt. Um das Risiko von unkontrollierbaren Blutungen zu reduzieren, sollte das "blutverdünnende Mittel" rechtzeitig vor dem Ereignis (Intervention) abgesetzt, d.h. nicht mehr genommen werden. In einem weiteren Schritt (103) wird demensprechend ein Absetzzeitpunkt ermittelt, nach dem das Arzneimittel aufgrund der Kontraindikation abgesetzt, d.h. nicht mehr eingenommen werden sollte. Es ist denkbar, dass ein Arzt dem Patienten den Absetzzeitpunkt mitteilt. Denkbar ist auch, dass die Überwachungseinheit oder die Computereinheit den Absetzzeitpunkt errechnet. Es ist denkbar, dass der Absetzzeitpunkt in einen virtuellen Kalender der Überwachungseinheit oder der Computereinheit eingetragen wird. In einem weiteren Schritt (104) registriert die Überwachungseinheit einen Einnahmeversuch nach dem Absetzzeitpunkt. In einem weiteren Schritt (105) übermittelt die Überwachungseinheit oder die Computereinheit einen Warnhinweis an den Patienten, dass das Arzneimittel nicht mehr eingenommen werden sollte.

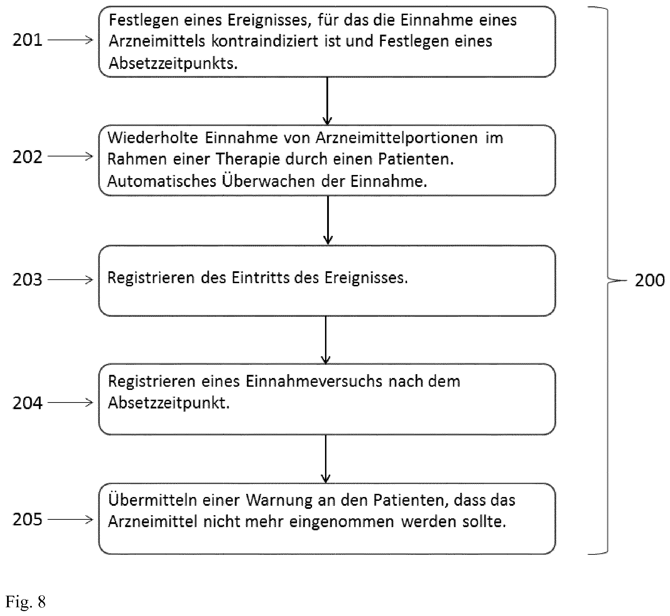

Figur 8 zeigt beispielhaft eine weitere Ausführungsform des erfindungsgemäßen Verfahrens (200), umfassend die Schritte (201), (202), (203), (204) und (205). In einem ersten Schritt (201) wird ein Ereignis festgelegt, für das die Einnahme eines Arzneimittels kontrainduziert ist, und es wird ein Absetzzeitpunkt festgelegt. Im vorliegenden Beispiel wird angenommen, dass der Absetzzeitpunkt mit dem Ereignis zeitlich zusammenfällt, d.h. nach Eintritt des Ereignisses sollte keine Arzneimittelportion mehr eingenommen werden. Zum Festlegen des Ereignisses zählt die Definition von einem oder mehreren Umständen, die erfüllt sein müssen, damit das Ereignis als eingetreten gilt. Denkbar ist zum Beispiel, dass ein Parameter einen definierten Grenzwert über- oder unterschritten hat. Bei dem Parameter könnte es sich um einen physiologischen Parameter des Patienten handeln, zum Beispiel um eine Konzentration eines Stoffes im Körper des Patienten. In einem weiteren Schritt (202) wird die wiederholte Einnahme von Arzneimittelportionen durch einen Patienten im Rahmen einer Therapie durch eine Überwachungseinheit automatisch überwacht; d.h. die Überwachungseinheit erkennt, ob der Patient eine Arzneimittelportion genommen hat oder ob der Patient einen Einnahmeversuch unternommen hat. In einem weiteren Schritt (203) wird der Eintritt des Ereignisses registriert. Es sind also diejenigen Umstände eingetreten, die in Schritt (201) definiert worden sind. Mittels der Überwachungseinheit wird in einem weiteren Schritt (204) ein Einnahmeversuch einer Arzneimittelportion durch den Patienten nach dem Absetzzeitpunkt registriert. In einem weiteren Schritt (205) übermittelt die Überwachungseinheit oder eine Computereinheit eine Warnung an den Patienten, dass das Arzneimittel nicht mehr eingenommen werden sollte.

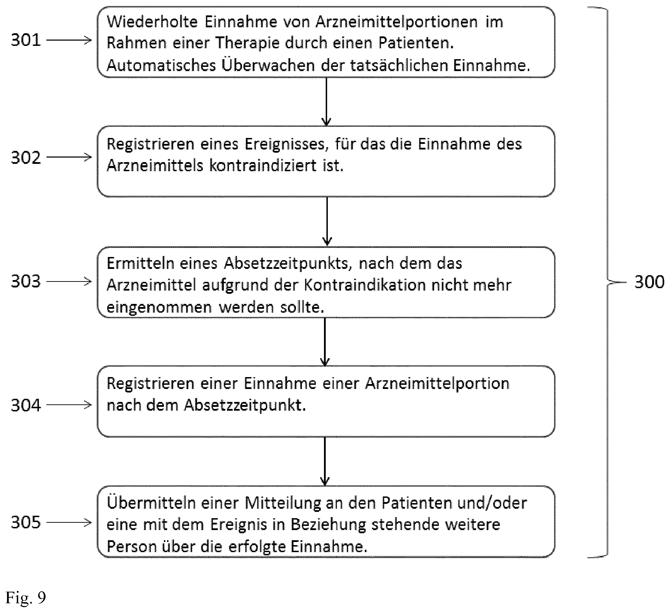

Figur 9 zeigt beispielhaft eine weitere Ausführungsform des erfindungsgemäßen Verfahrens (300), umfassend die Schritte (301), (302), (303), (304) und (305). Ein erster Schritt (301) symbolisiert die wiederholte Einnahme von Arzneimittelportionen durch einen Patienten im Rahmen einer Therapie. Der Patient wird dabei durch eine Überwachungseinheit unterstützt; die Überwachungseinheit registriert die tatsächliche Einnahme der Arzneimittelportionen durch den Patienten. In einem weiteren Schritt (302) wird ein Ereignis registriert, für das die weitere Einnahme des Arzneimittels kontraindiziert ist. Es ist denkbar, dass der Patient das Ereignis in einem virtuellen Kalender einträgt. In einem weiteren Schritt (303) wird ein Absetzzeitpunkt ermittelt, nach dem das Arzneimittel aufgrund der Kontraindikation abgesetzt, d.h. nicht mehr eingenommen werden sollte. Es ist denkbar, dass der Absetzzeitpunkt in einen virtuellen Kalender eingetragen wird. In einem weiteren Schritt (304) registriert die Überwachungseinheit die tatsächliche Einnahme einer Arzneimittelportion durch den Patienten nach dem Absetzzeitpunkt. In einem weiteren Schritt (304) übermittelt die Überwachungseinheit oder eine Computereinheit eine Mitteilung an den Patienten und/oder eine mit dem Ereignis in Beziehung stehende weitere Person über die erfolgte Einnahme.

**Patentansprüche**

**1.** Verfahren umfassend die Schritte

• automatisches Überwachen der wiederholten Einnahme eines Arzneimittels durch einen Patienten
• Registrieren eines Ereignisses, für das die Einnahme des Arzneimittels kontraindiziert ist
• Ermitteln eines Absetzzeitpunkts, nach dem das Arzneimittel aufgrund der Kontraindikation nicht mehr eingenommen werden sollte, **dadurch gekennzeichnet dass** das Verfahren außerdem umfasst:

> • Registrieren einer versuchten oder erfolgten Einnahme des Arzneimittels durch den Patienten selbst nach dem Absetzzeitpunkt
> • im Falle der versuchten Einnahme: Übermitteln einer Warnung an den Patienten, dass das Arzneimittel nicht eingenommen werden sollte
> • im Falle einer erfolgten Einnahme: Übermitteln einer Mitteilung an den Patienten und/oder eine mit dem Ereignis in Beziehung stehende weitere Person über die erfolgte Einnahme.

2. Verfahren gemäß Anspruch 1, wobei eine Überwachungseinheit, registriert,

   - wann vorbereitende Maßnahmen zur Einnahme von Arzneimittelportionen unternommen werden und/oder
   - wann der Patient tatsächlich Arzneimittelportionen einnimmt.

3. Verfahren gemäß einem der Ansprüche 1 oder 2, wobei der Patient die Überwachungseinheit als mobiles Gerät oder Bestandteil eines mobilen Geräts zur Erhöhung der Adhärenz zusammen mit Arzneimittelportionen mit sich führt.

4. Verfahren nach einem der Ansprüche 1 bis 3, wobei es sich bei dem Ereignis um ein für die Zukunft geplantes Ereignis handelt und der Absetzzeitpunkt zeitlich vor dem Zeitpunkt des für die Zukunft geplanten Ereignisses liegt oder mit diesem zusammenfällt.

5. Verfahren nach einem der Ansprüche 1 bis 4, wobei der Absetzzeitpunkt nach der Formel:

$$\text{Absetzzeitpunkt} = \text{Zeitpunkt des für die Zukunft geplanten Ereignisses} - \text{Wirkzeitspanne einer Arzneimittelportion}$$

errechnet wird.

6. Verfahren nach einem der Ansprüche 1 bis 5, wobei es sich bei dem Ereignis um eine medizinische Intervention am Körper des Patienten handelt, die mit Blutungen oder dem Risiko von Blutungen verbunden ist, und es sich bei dem Arzneimittel um ein Antikoagulans oder einen Thrombozytenfunktionshemmer handelt.

7. Verfahren nach einem der Ansprüche 1 bis 6, wobei eine tatsächliche Einnahme registriert wird und das für die Zukunft geplante Ereignis in Folge der Einnahme verschoben oder abgesagt wird oder Maßnahmen getroffen werden, um die zum Zeitpunkt des Ereignisses bestehende Wirkung des Arzneimittels zu verhindern oder zu reduzieren oder zu kompensieren.

8. Verfahren nach Anspruch einem der Ansprüche 1 bis 7, wobei das Ereignis **dadurch gekennzeichnet ist, dass** ein Parameter im Körper des Patienten oder einem Körperprodukt des Patienten einen definierten Wert annimmt oder einen Wert ober- oder unterhalb eines Schwellenwerts erreicht.

9. System umfassend

   • Mittel zum automatischen Überwachen der wiederholten Einnahme eines Arzneimittels durch einen Patienten
   • Mittel zum Registrieren eines Ereignisses, für das die Einnahme des Arzneimittels kontraindiziert ist
   • Mittel zum Ermitteln eines Absetzzeitpunkts, nach dem das Arzneimittel aufgrund der Kontraindikation nicht mehr eingenommen werden sollte, **dadurch gekennzeichnet, dass** das System außerdem Folgendes umfasst:

   > • Mittel zum Registrieren einer versuchten oder erfolgten Einnahme des Arzneimittels durch den Patienten selbst nach dem Absetzzeitpunkt
   > • Mittel zum Übermitteln einer Warnung an den Patienten, dass das Arzneimittel nicht eingenommen werden sollte und/oder zum Übermitteln einer Mitteilung an den Patienten und/oder eine mit dem Ereignis in Beziehung stehende weitere Person über die erfolgte Einnahme.

10. System nach Anspruch 9, umfassend eine Überwachungseinheit und eine Computereinheit, wobei die Überwachungseinheit so konfiguriert ist, dass sie einen Einnahmeversuch oder eine tatsächliche Einnahme einer Arzneimittelportion durch eine Person erkennt und Informationen über den Einnahmeversuch oder die tatsächliche Einnahme an die Computereinheit übermittelt, wobei die Computereinheit so konfiguriert ist, dass sie Informationen über den Einnahmeversuch oder die tatsächliche Einnahme von der Überwachungseinheit empfängt, und, falls der Einnahmeversuch oder die tatsächliche Einnahme zeitlich nach dem Absetzzeitpunkt erfolgt, ein Signal an den Nutzer der Computereinheit übermittelt.

11. System nach Anspruch 10, wobei Überwachungseinheit und Computereinheit zwei separate mobile Geräte sind.

12. System nach Anspruch 11, wobei Überwachungseinheit und Computereinheit zwei Bestandteile eines einzigen mobilen Geräts sind.

13. System nach einem der Ansprüche 10 bis 12, ferner umfassend einen Sensor, der einen Parameter im oder am Körper eines Patienten misst und oder einen Parameter in einem Körperprodukt misst und so konfiguriert ist, dass er gemessenen Werte an die Überwachungseinheit oder die Computereinheit übermittelt.

14. System nach einem der Ansprüche 10 bis 13, **dadurch gekennzeichnet, dass** das System eine Sperrfunktion aufweist, die aktiviert wird, sobald ein Einnahmeversuch nach dem Absetzzeitpunkt registriert wird, wobei die Sperrfunktion eine Entnahme einer Arzneimittelportion durch eine Person verhindert.

15. Computerprogrammprodukt umfassend einen Datenträger und Programmcode, der auf dem Datenträger gespeichert ist, und eine Computereinheit, in dessen Arbeitsspeicher der Programmcode geladen ist, dazu veranlasst, die folgenden Schritte auszuführen:

   • Empfangen eines Ereignis-Zeitpunkts für ein Ereignis, für das die Einnahme eines Arzneimittels kontraindiziert ist
   • Ermitteln eines Absetzzeitpunkts, ab dem das Arzneimittel aufgrund der Kontraindikation nicht mehr eingenommen werden sollte, **dadurch gekennzeichnet dass** das Computerprogrammprodukt außerdem so veranlasst ist, die folgenden Schritte auszuführen:

   • Registrieren eines Einnahmeversuchs oder eine erfolgten Einnahme einer Portion des Arzneimittels durch einen Patienten selbst nach dem Absetzzeitpunkt
   • Übermitteln einer Warnung an den Patienten, dass die Arzneimittelportion nicht eingenommen werden sollte, und/oder an eine mit dem Ereignis in Beziehung stehende weitere Person über die erfolgte Einnahme

wobei die Computereinheit umfasst: eine Eingabeeinheit, eine Verarbeitungseinheit umfassend den Arbeitsspeicher und einen Prozessor, und eine Ausgabeeinheit.

**Claims**

1. Method, comprising the steps of

   • automated monitoring of repeated taking of medication by a patient
   • registering an event for which taking the medication is contraindicated
   • identifying a discontinuation time after which the medication should no longer be taken on the basis of the contraindication, **characterized in that** the method furthermore comprises:
   • registering an attempt to take or an actual taking of the medication by the patient themselves after the discontinuation time
   • in the event of an attempt to take the medication: sending a warning to the patient that the medication should not be taken
   • in the event of an actual taking of the medication: sending a message informing the patient and/or another person related to the event about the actual taking thereof.

2. Method according to Claim 1, wherein a monitoring unit registers

   - when preparatory measures are performed for taking doses of medication and/or

- when the patient actually takes the doses of medication.

3. Method according to either of Claims 1 and 2, wherein the patient carries the monitoring unit on their person together with doses of medication, as a mobile device or component of a mobile device in order to increase adherence.

4. Method according to one of Claims 1 to 3, wherein the event is an event scheduled in the future and the discontinuation time occurs prior to, or coincides with, the time of the event scheduled in the future.

5. Method according to one of Claims 1 to 4, wherein the discontinuation time is calculated according to the formula:

$$\texttt{discontinuation time = time of the event scheduled in}$$
$$\texttt{the future - effective duration of a medication dosage.}$$

6. Method according to one of Claims 1 to 5, wherein the event is a medical intervention on the body of the patient, which is associated with bleeding or the risk of bleeding, and the medication is an anticoagulant or platelet function inhibitor.

7. Method according to one of Claims 1 to 6, wherein an actual taking of the medication is registered and the event scheduled in the future is postponed or cancelled as a result of the taking thereof, or measures are taken to prevent or reduce or compensate for the effect of the medication existing at the time of the event.

8. Method according to one of Claims 1 to 7, wherein the event is **characterized in that** a parameter in the patient's body or a bodily product of the patient assumes a defined value, or reaches a value above or below a threshold value.

9. System, comprising

   • means for the automated monitoring of repeated taking of medication by a patient
   • means for registering an event for which taking the medication is contraindicated
   • means for identifying a discontinuation time after which the medication should no longer be taken on the basis of the contraindication, **characterized in that** the system furthermore comprises the following:

      • means for registering an attempt to take or an actual taking of the medication by the patient themselves after the discontinuation time
      • means for sending a warning to the patient that the medication should not be taken and/or for sending a message informing the patient and/or another person related to the event about the actual taking thereof.

10. System according to Claim 9, comprising a monitoring unit and a computer unit,

    wherein the monitoring unit is configured so as to detect an attempt to take or an actual taking of a dose of medication by a person and send information about the attempt to take or actual taking thereof to the computer unit,
    wherein the computer unit is configured in such a way that it receives information about the attempted or actual taking thereof from the monitoring unit, and if the attempt to take or actual taking thereof occurs after the discontinuation time, sends a signal to the user of the computer unit.

11. System according to Claim 10, wherein the monitoring unit and the computer unit are two separate mobile devices.

12. System according to Claim 11, wherein the monitoring unit and the computer unit are two components of a single mobile device.

13. System according to one of Claims 10 to 12, further comprising a sensor that measures a parameter in or on the body of a patient and/or measures a parameter in a bodily product, and is configured so that it sends measured values to the monitoring unit or the computer unit.

14. System according to one of Claims 10 to 13, **characterized in that** the system has a blocking function that is activated as soon as an attempt to take the medication is registered after the discontinuation time, wherein the blocking function prevents a dose of medication being taken by a person.

**15.** Computer program product, comprising a data carrier and program code that is stored on the data carrier, and that prompts a computer unit, in the working memory of which the program code is loaded, to execute the following steps:

- receiving an event time for an event for which taking a medication is contraindicated
- identifying a discontinuation time from which the medication should no longer be taken on the basis of the contraindication, **characterized in that** the computer program product is furthermore prompted to execute the following steps:

  - registering an attempt to take or an actual taking of a dose of the medication by a patient themselves after the discontinuation time
  - sending a warning to the patient that the dose of medication should not be taken, and/or to another person related to the event about the actual taking thereof, wherein the computer unit comprises: an input unit, a processing unit comprising the working memory and a processor, and an output unit.

**Revendications**

**1.** Procédé comprenant les étapes suivantes

- surveiller automatiquement la prise répétée d'un médicament par un patient
- enregistrer un événement pour lequel la prise du médicament est contre-indiquée
- déterminer un instant d'arrêt après lequel le médicament ne doit plus être pris en raison de la contre-indication,

**caractérisé en ce que** le procédé comprend également les étapes suivantes :

- enregistrer la tentative de prise ou la prise effective du médicament par le patient lui-même après l'instant d'arrêt
- en cas de tentative de prise : envoyer au patient une notification selon laquelle il ne faut pas prendre le médicament
- en cas de prise effective : envoyer au patient et/ou à une autre personne liée à l'événement une notification concernant la prise effective.

**2.** Procédé selon la revendication 1, une unité de surveillance enregistrant,

- le moment où des mesures préparatoires à la prise de doses de médicaments sont prises et/ou
- le moment où le patient prend effectivement des doses de médicament.

**3.** Procédé selon l'une des revendications 1 ou 2, le patient emportant, conjointement avec des doses de médicament, l'unité de surveillance sous la forme d'un appareil mobile ou d'une partie d'un appareil mobile afin d'augmenter l'observance thérapeutique.

**4.** Procédé selon l'une des revendications 1 à 3, l'événement étant un événement futur planifié et l'instant d'arrêt étant antérieur à l'instant de l'événement futur planifié ou coïncidant avec celui-ci.

**5.** Procédé selon l'une des revendications 1 à 4, l'instant d'arrêt étant calculé selon la formule :
Instant d'arrêt = instant de l'événement futur planifié - durée d'action d'une dose de médicament.

**6.** Procédé selon l'une des revendications 1 à 5, l'événement étant une intervention médicale sur le corps du patient associée à des saignements ou à un risque de saignements et le médicament étant un anticoagulant ou un antiagrégant plaquettaire.

**7.** Procédé selon l'une des revendications 1 à 6, une prise effective étant enregistrée et l'événement futur planifié étant reporté ou annulé du fait de la prise ou des mesures étant prises pour prévenir ou réduire ou compenser l'effet du médicament à l'instant de l'événement.

**8.** Procédé selon l'une des revendications 1 à 7, l'événement étant **caractérisé en ce qu'**un paramètre dans le corps du patient ou un produit corporel du patient prend une valeur définie ou atteint une valeur supérieure ou inférieure à une valeur seuil.

9.  Système comprenant

    • des moyens pour surveiller automatiquement la prise répétée d'un médicament par un patient
    • des moyens pour enregistrer un événement pour lequel la prise du médicament est contre-indiquée
    • des moyens pour déterminer un instant d'arrêt après lequel le médicament ne doit plus être pris en raison de la contre-indication,

    **caractérisé en ce que** le système comprend également :

    • des moyens pour enregistrer la tentative de prise ou la prise effective du médicament par le patient même après l'instant d'arrêt
    • des moyens pour envoyer au patient une notification selon laquelle le médicament ne doit pas être pris et/ou pour envoyer au patient et/ou à une autre personne associée à l'événement une notification selon laquelle le médicament a été pris.

10. Système selon la revendication 9, comprenant une unité de surveillance et une unité informatique,

    l'unité de surveillance étant configurée de manière à reconnaître une tentative de prise ou une prise effective d'une dose de médicament par une personne et envoyer des informations sur la tentative de prise ou la prise effective à l'unité informatique,
    l'unité informatique étant configurée de manière à recevoir des informations sur la tentative de prise ou la prise effective de l'unité de surveillance et, si la tentative de prise ou la prise réelle se produit après l'instant d'arrêt, envoyer un signal à l'utilisateur de l'unité informatique.

11. Système selon la revendication 10, l'unité de surveillance et l'unité informatique étant deux appareils mobiles distincts.

12. Système selon la revendication 11, l'unité de surveillance et l'unité informatique étant deux composants d'un même appareil mobile.

13. Système selon l'une des revendications 10 à 12, comprenant en outre un capteur qui mesure un paramètre dans ou sur le corps d'un patient et/ou un paramètre dans un produit corporel et étant configuré pour envoyer des valeurs mesurées à l'unité de surveillance. ou à l'unité informatique.

14. Système selon l'une des revendications 10 à 13, **caractérisé en ce que** le système comporte une fonction de blocage qui est activée dès qu'une tentative de prise est enregistrée après l'instant d'arrêt, la fonction de blocage empêchant une personne de prendre une dose de médicament.

15. Progiciel comprenant un support de données et un code de programme, qui est stocké sur le support de données, et ordonnant à une unité informatique, dans la mémoire de travail de laquelle le code de programme est chargé, d'exécuter les étapes suivantes :

    • recevoir un instant d'événement relatif à un événement pour lequel la prise d'un médicament est contre-indiquée
    • déterminer un instant d'arrêt à partir duquel le médicament ne doit plus être pris en raison de la contre-indication,

    **caractérisé en ce que** le progiciel ordonne également d'exécuter les étapes suivantes :

    • enregistrer une tentative de prise ou une prise effective d'une dose de médicament par un patient lui-même après l'instant d'arrêt
    • envoyer au patient une notification selon laquelle la dose de médicament ne doit pas être prise et/ou à une autre personne associée à l'événement une notification concernant la prise effective

    l'unité informatique comprenant : une unité d'entrée, une unité de traitement comprenant la mémoire de travail et un processeur, et une unité de sortie.

Fig. 1

Fig. 2

1

25

10

30

Fig.3

39

32

30

35

37

36

38

33a

33

33b

31

Fig. 4

Fig. 5

Fig. 6

101 ⟶ Wiederholte Einnahme von Arzneimittelportionen im Rahmen einer Therapie durch einen Patienten. Eine Überwachungseinheit registriert dabei die Entnahmen der Arzneimittelportionen aus einem Behälter.

102 ⟶ Registrieren eines Ereignisses, für das die Einnahme des Arzneimittels kontraindiziert ist.

103 ⟶ Ermitteln eines Absetz-Zeitpunkts, nach dem das Arzneimittel aufgrund der Kontraindikation nicht mehr eingenommen werden sollte.

100

104 ⟶ Registrieren eines Einnahmeversuchs nach dem Absetz-Zeitpunkt.

105 ⟶ Übermitteln einer Warnung an den Patienten, dass das Arzneimittel nicht mehr eingenommen werden sollte.

Fig. 7

201 ——→ Festlegen eines Ereignisses, für das die Einnahme eines Arzneimittels kontraindiziert ist und Festlegen eines Absetzzeitpunkts.

202 ——→ Wiederholte Einnahme von Arzneimittelportionen im Rahmen einer Therapie durch einen Patienten. Automatisches Überwachen der Einnahme.

203 ——→ Registrieren des Eintritts des Ereignisses.

200

204 ——→ Registrieren eines Einnahmeversuchs nach dem Absetzzeitpunkt.

205 ——→ Übermitteln einer Warnung an den Patienten, dass das Arzneimittel nicht mehr eingenommen werden sollte.

Fig. 8

301 ——→ Wiederholte Einnahme von Arzneimittelportionen im Rahmen einer Therapie durch einen Patienten. Automatisches Überwachen der tatsächlichen Einnahme.

302 ——→ Registrieren eines Ereignisses, für das die Einnahme des Arzneimittels kontraindiziert ist.

303 ——→ Ermitteln eines Absetzzeitpunkts, nach dem das Arzneimittel aufgrund der Kontraindikation nicht mehr eingenommen werden sollte.

} — 300

304 ——→ Registrieren einer Einnahme einer Arzneimittelportion nach dem Absetzzeitpunkt.

305 ——→ Übermitteln einer Mitteilung an den Patienten und/oder eine mit dem Ereignis in Beziehung stehende weitere Person über die erfolgte Einnahme.

Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2013035951 A
- WO 9604881 A1

- DE 19516076 A1